# EUROPEAN PATENT SPECIFICATION

(11) **EP 4 664 343 B1**
(45) Date of publication and mention of the grant of the patent: **18.11.2026**
(21) Application number: 24875732.0
(22) Date of filing: 01.07.2024
(51) Int. Cl.: G06F 30/20, G06F 119/14, G06F 111/10, G01N 3/08, H01M 4/04, H01M 10/42

(54) **METHOD AND APPARATUS FOR CALIBRATING CONTACT PARAMETERS OF ELECTRODE MATERIAL, AND COMPUTER STORAGE MEDIUM**
VERFAHREN UND VORRICHTUNG ZUR KALIBRIERUNG VON KONTAKTPARAMETERN VON ELEKTRODENMATERIAL UND COMPUTERSPEICHERMEDIUM
PROCÉDÉ ET APPAREIL D'ÉTALONNAGE DE PARAMÈTRES DE CONTACT D'UN MATÉRIAU D'ÉLECTRODE, ET SUPPORT DE STOCKAGE INFORMATIQUE

(30) Priority: 30.04.2024 CN 202410547629
(43) Date of publication of application: 17.12.2025
(73) Proprietor: Eve Energy Co., Ltd., Huizhou, Guangdong 516000 (CN)
(72) Inventor: LIAO, Qianyan, Huizhou, Guangdong 516000 (CN); CHEN, Zebin, Huizhou, Guangdong 516000 (CN); CAO, Lang, Huizhou, Guangdong 516000 (CN); LIU, Yifan, Huizhou, Guangdong 516000 (CN); ZHAI, Mo, Huizhou, Guangdong 516000 (CN)
(74) Representative: HGF
(86) International application number: PCT/CN2024/102948
(87) International publication number: WO 2025/227491

(56) References cited:
- WO-A1-2023/133732
- CN-A- 113 887 105
- CN-A- 115 579 085
- CN-A- 116 068 406
- SCHREINER DAVID ET AL: "DEM Simulations of the Calendering Process: Parameterization of the Electrode Material of Lithium-Ion Batteries", PROCEDIA CIRP, vol. 104, 1 January 2021 (2021-01-01), NL, pages 91 - 97, XP093361530, ISSN: 2212-8271, DOI: 10.1016/j.procir.2021.11.016
- SANGRÓS GIMÉNEZ CLARA ET AL: "Numerical simulation of the behavior of lithium-ion battery electrodes during the calendaring process via the discrete element method", POWDER TECHNOLOGY, vol. 349, 1 May 2019 (2019-05-01), pages 1 - 11, XP085668761, ISSN: 0032-5910, DOI: 10.1016/J.POWTEC.2019.03.020
- RACKL MICHAEL ET AL: "A methodical calibration procedure for discrete element models", POWDER TECHNOLOGY, vol. 307, 1 February 2017 (2017-02-01), pages 73 - 83, XP029863024, ISSN: 0032-5910, DOI: 10.1016/J.POWTEC.2016.11.048
- BU PENG ET AL: "A calibration method of discrete element contact model parameters for bulk materials based on experimental design method", POWDER TECHNOLOGY, vol. 425, 1 July 2023 (2023-07-01), Basel (CH), pages 118596, XP093361446, ISSN: 0032-5910, DOI: 10.1016/j.powtec.2023.118596

## Description

### TECHNICAL FIELD

The present disclosure relates to the technical field of electrode material parameter calibration and, in particular, to a method and apparatus for calibrating contact parameters of electrode materials, and a computer storage medium.

### BACKGROUND

In the preparation of positive electrodes for liquid cathode lithium batteries, for an integrated carbon-encapsulated positive electrode, powders or particles powders or particles with consistent porosity and particle diameter are acquired first through processing steps such as mixing, fiber-forming, and sieving. Then the powders or particles are pressed, slit, or directly extruded to obtain a positive electrode plate. However, such a positive electrode preparation process easily leads to phenomena including loose powders or particles and uneven compacted density in local regions, thereby affecting the performance of a battery.

At present, an important method for simulating values of powder/bulk materials, that is, a discrete element method (DEM), is generally used to optimize the design of key components of a liquid cathode lithium battery and discover the setting basis of parameters of the electrode plate molding process.

At present, in the process of acquiring discrete element simulation parameters, it is difficult to directly measure contact parameters such as stiffness, shear modulus, friction coefficient, and other model parameters. Moreover, the relatively low correlation between the existing model parameter calibration methods and the application of electrode materials makes the accuracy of parameter calibration relatively low, which is not conducive to further research on the preparation process of electrode materials. Document XP093361530 represents relevant prior art.

### SUMMARY

The invention is defined by the claims. In a first aspect, the present disclosure provides a method for calibrating contact parameters of electrode materials. The method includes the steps below.

An axial force-displacement curve of a target pellet of an electrode material is acquired; and whether the electrode material meets an adaptation condition of a preset contact model is judged according to the axial force-displacement curve. The axial force-displacement curve is obtained by performing a uniaxial unconfined compression test on the target pellet.

When the electrode material meets the adaptation condition of the preset contact model, calibration ranges of a plurality of contact parameters of the electrode material are determined; and a discrete element simulation test is performed on the electrode material based on the calibration ranges of all the contact parameters and a preset uniaxial confined compression calibration model to obtain a target discrete element simulation parameter of the electrode material. The uniaxial confined compression calibration model is established based on the contact model and an actual application scenario of the electrode material.

A uniaxial confined compression simulation test is performed on the electrode material according to the target discrete element simulation parameter to obtain a simulated axial pressure-axial strain relationship of the electrode material; whether all the plurality of contact parameters are calibrated successfully is judged according to the simulated axial pressure-axial strain relationship, a pre-obtained measured axial pressure-axial strain relationship of the electrode material, and a measured compacted density parameter; and when all the plurality of contact parameters are calibrated successfully, calibration results of all the plurality of contact parameters are determined according to the target discrete element simulation parameter.

In a second aspect, the present disclosure provides an apparatus for calibrating contact parameters of electrode materials. The apparatus includes an acquisition module, a judgment module, a determination module, a first simulation test module, and a second simulation test module.

The acquisition module is configured to acquire an axial force-displacement curve of a target pellet of an electrode material.

The judgment module is configured to judge, according to the axial force-displacement curve, whether the electrode material meets an adaptation condition of a preset contact model, where the axial force-displacement curve is obtained by performing a uniaxial unconfined compression test on the target pellet.

The determination module is configured to determine calibration ranges of a plurality of contact parameters of the electrode material when the electrode material meets the adaptation condition of the preset contact model.

The first simulation test module is configured to perform a discrete element simulation test on the electrode material based on the calibration ranges of all the plurality of contact parameters and a preset uniaxial confined compression calibration model to obtain a target discrete element simulation parameter of the electrode material, where the uniaxial confined compression calibration model is established based on the contact model and an actual application scenario of the electrode material.

The second simulation test module is configured to perform a uniaxial confined compression simulation test on the electrode material according to the target discrete element simulation parameter to obtain a simulated axial pressure-axial strain relationship of the electrode material.

The judgment module is further configured to judge whether all the plurality of contact parameters are calibrated successfully according to the simulated axial pressure-axial strain relationship, a pre-obtained measured axial pressure-axial strain relationship of the electrode material, and a measured compacted density parameter.

The determination module is further configured to determine calibration results of all the plurality of contact parameters according to the target discrete element simulation parameter when all the plurality of contact parameters are calibrated successfully.

In a third aspect, the present disclosure provides an apparatus for calibrating contact parameters of electrode materials. The apparatus includes a memory storing executable program codes and a processor coupling with the memory.

The processor calls the executable program codes stored in the memory to perform the method for calibrating contact parameters of an electrode material disclosed in the first aspect of the present disclosure.

In a fourth aspect, the present disclosure provides a computer storage medium for storing computer instructions, where when the computer instructions are called, the method for calibrating contact parameters of an electrode material disclosed in the first aspect of the present disclosure is performed.

In the present disclosure, a discrete element simulation test is performed on an electrode material based on a calibration model and calibration ranges of contact parameters of the electrode material when an axial force-displacement curve of a target pellet of the electrode material meets an adaption condition of the contact model; then a simulated axial pressure-axial strain relationship of the electrode material is acquired based on a target discrete element simulation parameter of the electrode material; whether the contact parameters are calibrated successfully is judged based on this relationship; and when the contact parameters are calibrated successfully, calibration results of the contact parameters are determined. It can be seen that the present disclosure can be implemented through adopting a test-simulation joint calibration method to calibrate the contact parameters of the electrode material. Such an arrangement simplifies the testing process and improves the reliability and accuracy of calibrating the contact parameters of the electrode material compared with the direct measurement method, thereby providing a good theoretical basis for the preparation process of the electrode material.

### BRIEF DESCRIPTION OF DRAWINGS

FIG. 1 is a flowchart of a method for calibrating contact parameters of electrode materials according to an embodiment of the present disclosure.
FIG. 2 is another flowchart of the method for calibrating contact parameters of electrode materials according to an embodiment of the present disclosure.
FIG. 3 is a structural diagram of an apparatus for calibrating contact parameters of electrode materials according to an embodiment of the present disclosure.
FIG. 4 is another structural diagram of the apparatus for calibrating contact parameters of electrode materials according to an embodiment of the present disclosure.

### DETAILED DESCRIPTION

The present disclosure discloses a method and apparatus for calibrating contact parameters of electrode materials, and a computer storage medium, which simplifies the testing process and improves the reliability and accuracy of calibrating contact parameters of an electrode material compared with the direct measurement method, thereby providing a good theoretical basis for the preparation process of the electrode material.

### Embodiment one

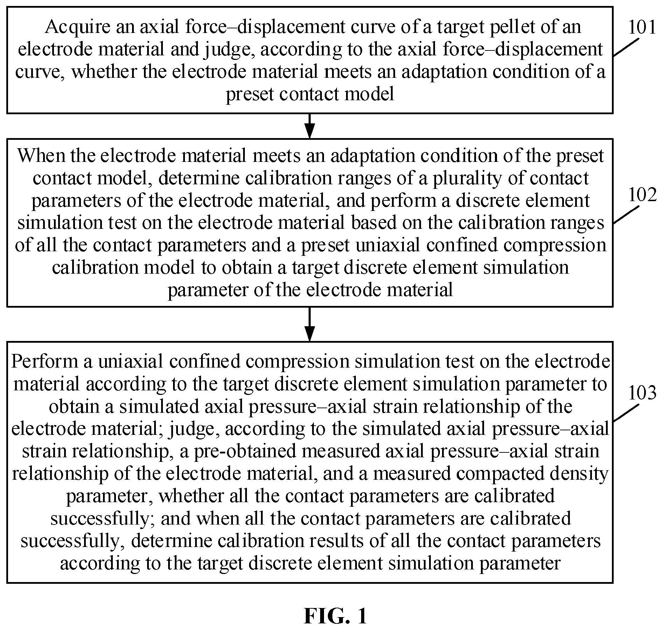

Referring to FIG. 1, FIG. 1 is a flowchart of a method for calibrating contact parameters of an electrode material according to an embodiment of the present disclosure. In some implementations, the method may be implemented by an apparatus for calibrating contact parameters. The apparatus for calibrating contact parameters may be integrated into a device for calibrating contact parameters, such as an intelligent computer and a related electrode material preparation device. When the apparatus for calibrating contact parameters exists independently, it may also be, for example, a local server or a cloud server for processing the process of calibrating contact parameters of electrode materials. This is not limited in the embodiment of the present disclosure. As shown in FIG. 1, the method for calibrating contact parameters of an electrode material may include the operations below.

In 101, an axial force-displacement curve of a target pellet of an electrode material is acquired, and whether the electrode material meets an adaptation condition of a preset contact model is judged according to the axial force-displacement curve.

In the embodiment of the present disclosure, the axial force-displacement curve is obtained by performing a uniaxial unconfined compression test on the target pellet. For example, firstly, the electrode material is pressed (for example, into a cylindrical test block with an inner diameter of x mm and a height of y mm) to obtain the target pellet; then the universal mechanical testing machine is set to push a pressure plate downwards in the axial direction at a constant speed; when the axial stress applied to the pressure plate reaches a preset pressure value, the pressure plate returns to the original position at the same speed; finally, the pressure plate is furthered downwards (that is, repeat above operations) until the target pellet is damaged. Moreover, the axial force-displacement curve in the two processes is recorded so as to analyze the deformation condition (for example, plastic deformation or elastic deformation) of the target pellet of the electrode material according to the axial force-displacement curve, thereby determining the matching between the electrode material and the preset contact model.

In 102, the electrode material meets the adaptation condition of the preset contact model, calibration ranges of a plurality of contact parameters of the electrode material are determined, and a discrete element simulation test is performed on the electrode material based on the calibration ranges of all the contact parameters and a preset uniaxial confined compression calibration model to obtain a target discrete element simulation parameter of the electrode material.

In the embodiment of the present disclosure, the uniaxial confined compression calibration model is established based on the contact model and an actual application scenario of the electrode material, such as a Hysteretic Spring model or a Hertz-Mindlin (no slip) model. In some implementations, the calibration ranges of all the contact parameters of the electrode material may be determined through the tapping method and the sieving method.

In some implementations, before the discrete element simulation test is performed on the electrode material based on the calibration ranges of all the contact parameters and the preset uniaxial confined compression calibration model to obtain the target discrete element simulation parameter of the electrode material, a uniaxial confined compression test may be performed on the electrode material first. For example, after the container is filled with the particle material of the electrode material, the pressure plate is loaded at a constant rate with the adoption of the universal testing machine. When the displacement reaches the preset displacement parameter, the pressure plate returns. Tests are repeated multiple times. The axial force and displacement data in each testing process is recorded so that a measured axial pressure-axial strain relationship (for example, a measured axial pressure-axial strain curve where y = a · x^{b}, specific values of coefficients a and b, and a measured compacted density parameter) of the electrode material is obtained through analysis. In this case, the measured axial pressure-axial strain relationship of the electrode material may be compared with a simulated axial pressure-axial strain relationship of the electrode material subsequently to judge whether the contact parameters of the electrode material are calibrated successfully.

In some implementations, the setting process of the uniaxial confined compression calibration model may be as follows: In the simulation test, a cylinder with the same dimension as an actual cylindrical tube is used to simulate the actual cylindrical tube; then a spherical particle with a corresponding diameter is used to simulate a sponge-like porous carbon cathode particle; a particle workshop is set according to the particle diameter distribution; and particles with the same mass as actual particles are generated to fill the cylinder. Therefore, the setting of the uniaxial confined compression calibration model is completed.

In 103, a uniaxial confined compression simulation test is performed on the electrode material according to the target discrete element simulation parameter to obtain a simulated axial pressure-axial strain relationship of the electrode material; whether all the contact parameters are calibrated successfully is judged according to the simulated axial pressure-axial strain relationship, a pre-obtained measured axial pressure-axial strain relationship of the electrode material, and a measured compacted density parameter; and when all the plurality of contact parameters are calibrated successfully, calibration results of all the contact parameters are determined according to the target discrete element simulation parameter.

In the embodiment of the present disclosure, in some implementations, the method further includes the following: When it is judged that all the contact parameters are not calibrated successfully, a model parameter of the uniaxial confined compression calibration model may be adjusted to update the uniaxial confined compression calibration model, thereby triggering the operation in the preceding step 102 that the discrete element simulation test is performed on the electrode material based on the calibration ranges of all the contact parameters and the preset uniaxial confined compression calibration model to obtain the target discrete element simulation parameter of the electrode material.

It can be seen that the embodiment of the present disclosure can be implemented through adopting a test-simulation joint calibration method to calibrate the contact parameters of the electrode material. Such an arrangement simplifies the testing process and improves the reliability and accuracy of calibrating the contact parameters of the electrode material compared with the direct measurement method, thereby providing a good theoretical basis for the preparation process of the electrode material.

In some implementations, the preceding step 102 in which the calibration ranges of the contact parameters of the electrode material are determined includes the steps below.

A particle density parameter of the electrode material is determined, and a particle diameter distribution parameter of the electrode material is determined.

The calibration ranges of the contact parameters of the electrode material are determined according to the particle density parameter and the particle diameter distribution parameter.

In some implementations, the particle density parameter of the electrode material may be obtained based on the tapping method. The particle diameter distribution parameter includes a particle diameter parameter and a particle proportion parameter corresponding to the particle diameter parameter. For example, the particle proportion of a particle with a diameter of 1.5 mm is measured to be 0.25% based on the sieving method. In some implementations, all the contact parameters include at least one of an inter-particle recovery coefficient, an inter-particle static friction coefficient, an inter-particle rolling friction coefficient, a shear modulus coefficient, a damping coefficient, a stiffness factor parameter, or a yield strength parameter. The shear modulus coefficient, the damping coefficient, the stiffness factor parameter, and the yield strength parameter are used for indicating the shear modulus coefficient, damping coefficient, stiffness factor parameter and yield strength parameter between a particle and its container wall respectively.

It can be seen that in the preceding implementations, the particle density parameter of the electrode material and the particle diameter distribution parameter of the electrode material can be measured based on the tapping method and the sieving method. Then the calibration ranges of the contact parameters of the electrode material can be determined. Such an arrangement can provide a reliable simulation parameter reference range for the subsequent discrete element simulation test, improving the reliability, accuracy and effectiveness of performing the discrete element simulation test, thereby improving the reliability and accuracy of comparing the subsequent test-simulation results, and thus improving the accuracy of judging whether the contact parameters of the electrode material are calibrated successfully.

### Embodiment two

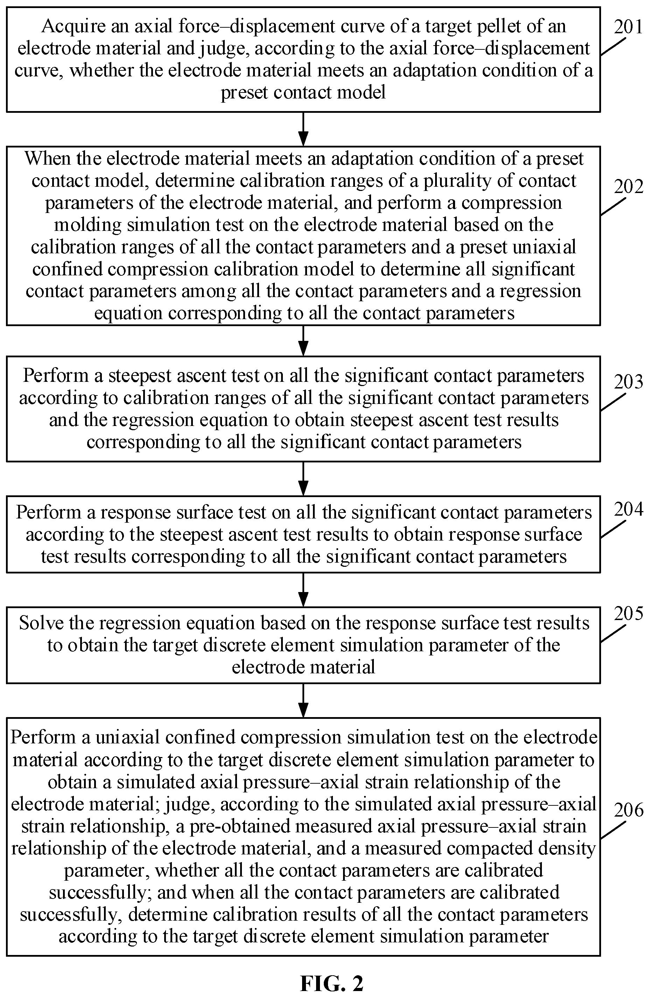

Referring to FIG. 2, FIG. 2 is another flowchart of the method for calibrating contact parameters of an electrode material according to an embodiment of the present disclosure. In some implementations, the method may be implemented by an apparatus for calibrating contact parameters. The apparatus for calibrating contact parameters may be integrated into a device for calibrating contact parameters, such as an intelligent computer and a related electrode material preparation device. When the apparatus for calibrating contact parameters exists independently, it may also be, for example, a local server or a cloud server for processing the process of calibrating contact parameters of an electrode material. This is not limited in the embodiment of the present disclosure. As shown in FIG. 2, the method for calibrating contact parameters of an electrode material may include the operations below.

In 201, an axial force-displacement curve of a target pellet of an electrode material is acquired, and whether the electrode material meets an adaptation condition of a preset contact model is judged according to the axial force-displacement curve.

In 202, when the electrode material meets THE adaptation condition of THE preset contact model, calibration ranges of a plurality of contact parameters of the electrode material are determined, and a compression molding simulation test is performed on the electrode material based on the calibration ranges of all the contact parameters and a preset uniaxial confined compression calibration model to determine all significant contact parameters among all the contact parameters and a regression equation corresponding to all the contact parameters.

In the embodiment of the present disclosure, in some implementations, to improve the accuracy of the error detection of the compression molding simulation test, the compression molding simulation test may be performed on the electrode material based on the calibration ranges of all the contact parameters and the preset uniaxial confined compression calibration model in combination with a preset number of virtual parameters.

In 203, a steepest ascent test is performed on all the significant contact parameters according to calibration ranges of all the significant contact parameters and the regression equation to obtain steepest ascent test results corresponding to all the significant contact parameters.

In the embodiment of the present disclosure, in some implementations, the most significant contact parameter among all the significant contact parameters may be taken as the climbing unit and the climbing gradient direction. Moreover, the steepest ascent test is performed on all the significant contact parameters according to the calibration ranges of all the significant contact parameters and the regression equation to obtain the steepest ascent test results corresponding to all the significant contact parameters.

In 204, a response surface test is performed on all the significant contact parameters according to the steepest ascent test results to obtain response surface test results corresponding to all the significant contact parameters.

In the embodiment of the present disclosure, in some implementations, the response surface test may be understood as studying the influence of, for example, a single variable, an interactive variable, or a square variable on a response index.

In 205, solving operation is performed on the regression equation based on the response surface test results to obtain the target discrete element simulation parameter of the electrode material.

In the embodiment of the present disclosure, in some implementations, a physical test value is taken as a target to solve the regression equation so that the optimal solution of each contact parameter of the electrode material is obtained and taken as the target discrete element simulation parameter of the electrode material.

In 206, a uniaxial confined compression simulation test is performed on the electrode material according to the target discrete element simulation parameter to obtain a simulated axial pressure-axial strain relationship of the electrode material; whether all the contact parameters are calibrated successfully is judged according to the simulated axial pressure-axial strain relationship, a pre-obtained measured axial pressure-axial strain relationship of the electrode material, and a measured compacted density parameter; and when all the contact parameters are calibrated successfully, calibration results of all the contact parameters are determined according to the target discrete element simulation parameter.

In the embodiment of the present disclosure, for other descriptions of step 201 and step 206, refer to detailed descriptions of step 101 and step 103 in embodiment one, which is not repeated in the embodiment of the present disclosure.

It can be seen that through implementing the embodiment of the present disclosure, the compression molding simulation test can be performed on the electrode material through the uniaxial confined compression calibration model to obtain the significant contact parameters of the electrode material and the regression equation corresponding to the contact parameters. Then the steepest ascent test and the response surface test can be performed on the significant contact parameters of the electrode material so as to solve the regression equation and obtain the optimal solutions of the contact parameters of the electrode material. Such an arrangement can guarantee the reliability and accuracy of the simulation result of the model and the operation stability of the model, thereby improving the calibration efficiency of the contact parameters of the electrode material and thus applying the calibration model to the preparation process of the electrode material.

In some implementations, the preceding step 202 in which the compression molding simulation test is performed on the electrode material based on the calibration ranges of all the contact parameters and the preset uniaxial confined compression calibration model to determine all the significant contact parameters among all the contact parameters and the regression equation corresponding to all the contact parameters includes the steps below.

The compression molding simulation test is performed on the electrode material based on the calibration ranges of all the contact parameters and the preset uniaxial confined compression calibration model by taking an axial strain rate of the electrode material as a response index to obtain an axial strain rate simulation parameter of the electrode material regarding all the contact parameters and the axial strain rate simulation parameter is served as a compression molding simulation result of the electrode material.

Parameter screening test significance analysis is performed on all the contact parameters according to the compression molding simulation result to obtain a significance parameter of each contact parameter; and all the significant contact parameters whose significance parameters are greater than or equal to a preset significance threshold are determined from all the contact parameters according to significance parameters of all the contact parameters.

The regression equation corresponding to all the contact parameters at the axial strain rate is determined according to the compression molding simulation result.

In some implementations, for example, a parameter screening test table is created. The axial strain rate is taken as the response index to perform two-level tests of n groups on three virtual parameters and seven contact parameters, that is, an inter-particle recovery coefficient A_X₁_e, an inter-particle static friction coefficient B_X₂_µs, an inter-particle rolling friction coefficient C_X₃_µr, a shear modulus coefficient D_X₄_G, a damping coefficient E_X₅_bn, a stiffness factor parameter F_X₆_Kn, and a yield strength parameter G_X₇_Yn to obtain the compression molding simulation result. In this case, the P value correlation coefficient R² of correlation coefficient R can be analyzed. When an analysis result shows that the correlation and reliability of the model are relatively sound, the significant contact parameters among all the contact parameters and the corresponding regression equation can be determined according to the compression molding simulation result.

It can be seen that in the preceding implementations, uniaxial confined compression can be taken as the calibration model and applied to the simulation of the compaction forming process of the electrode material. Then the significant contact parameters of the electrode material and the regression equation corresponding to the contact parameters are determined. Such an arrangement helps improve the reliability and accuracy of determining the significant contact parameters and the regression equation, thus improving the efficiency of performing the subsequent steepest ascent test on the significant contact parameters and obtaining the optimal solutions of the contact parameters accurately and in rapid convergence.

The preceding step 203 in which the steepest ascent test is performed on all the significant contact parameters according to the calibration ranges of all the significant contact parameters and the regression equation to obtain the steepest ascent test results corresponding to all the significant contact parameters includes the steps below.

Analysis is performed on an influence effect of the most significant contact parameter with the largest significance parameter at the axial strain rate according to the regression equation.

A climbing unit and a climbing gradient direction that correspond to each significant contact parameter are determined according to the influence effect of the most significant contact parameter and a calibration range corresponding to the most significant contact parameter.

The steepest ascent test is performed on all the significant contact parameters according to climbing units corresponding to all the significant contact parameters, climbing gradient directions corresponding to all the significant contact parameters, and the calibration ranges corresponding to all the significant contact parameters to obtain the steepest ascent test results corresponding to all the significant contact parameters.

In some implementations, the influence effect includes a negative effect or a positive effect. Specifically, in the steepest ascent test on all the significant contact parameters, median values of calibration ranges corresponding to other contact parameters whose significance parameters are less than a preset significance threshold can be taken. All the significant contact parameters are used as test variables to simulate the axial strain rate of the electrode material.

It can be seen that in the preceding implementations, the most significant contact parameter can be taken as the climbing unit and the climbing gradient direction so as to perform the steepest ascent test on all the significant contact parameters to obtain the steepest ascent test results corresponding to all the significant contact parameters. Such an arrangement helps improve the reliability and accuracy of performing the steepest ascent test on the significant contact parameters, thereby improving the reliability and accuracy of performing the subsequent response surface test on the significant contact parameters, and thus improving the performing solving operation on efficiency of the target discrete element simulation parameter of the electrode material.

In some implementations, the preceding step 204 in which the response surface test is performed on all the significant contact parameters according to the steepest ascent test results to obtain the response surface test results corresponding to all the significant contact parameters includes the steps below.

The measured axial pressure-axial strain relationship of the electrode material is acquired.

A response surface test parameter range corresponding to each significant contact parameter is determined according to the steepest ascent test results; and the response surface test is performed on all the significant contact parameters according to response surface test parameter ranges corresponding to all the significant contact parameters and preset variable research objects corresponding to all the significant contact parameters by taking the axial strain rate and the relationship value as response indexes to obtain the response surface test results corresponding to all the significant contact parameters.

In some implementations, the measured axial pressure-axial strain relationship is obtained by performing a uniaxial confined compression test (that is, a physical test) on the electrode material. In some implementations, the measured axial pressure-axial strain relationship includes a relationship value between the measured axial pressure and measured axial strain that are applied to the electrode material in the uniaxial confined compression test, for example, coefficients a and b in a measured axial pressure-axial strain curve where y = a · x^{b}). In some implementations, the variable research objects include at least one of a single variable research object, an interactive variable research object, or a square variable research object, for example, a single variable research object X₁ of an inter-particle recovery coefficient A_X₁_e, an interactive variable research object X₁ X₂ between the inter-particle recovery coefficient A_X₁_e and inter-particle static friction coefficient B_X₂_µs, or a square variable research object X₁² of the inter-particle recovery coefficient A_X₁_e.

In some implementations, in the response surface test on all the significant contact parameters, a test may be created with the significant contact parameters as independent variables. Values of other contact parameters are consistent with the steepest ascent test. In some other implementations, when the response surface test results corresponding to all the significant contact parameters are obtained, variance analysis may be conducted on the model used in the response surface test to guarantee the high goodness of fit and response stability of the model, thereby performing solving operation on the regression equation according to the response surface test results.

It can be seen that in the preceding implementations, the response surface test parameter ranges corresponding to the significant contact parameters can be determined according to the steepest ascent test results. Then the response surface test is performed on the significant contact parameters according to the response surface test parameter ranges corresponding to the significant contact parameters and the preset variable research objects corresponding to the significant contact parameters by taking the axial strain rate and the relationship value as the response indexes. Such an arrangement improves the reliability and accuracy of performing the response surface test on the significant contact parameters, thereby improving the efficiency of performing solving operation on the regression equation subsequently, and thus improving the efficiency and accuracy of calibrating the contact parameters of the electrode material.

In some implementations, the preceding step 206 in which whether all the contact parameters are calibrated successfully is judged according to the simulated axial pressure-axial strain relationship, the pre-obtained measured axial pressure-axial strain relationship of the electrode material, and the measured compacted density parameter includes the steps below.

A target difference parameter of the electrode material is calculated according to the simulated axial pressure-axial strain relationship, the pre-obtained measured axial pressure-axial strain relationship of the electrode material, and the measured compacted density parameter.

A simulation-measurement difference parameter of the electrode material is determined according to the target difference parameter; whether the simulation-measurement difference parameter is less than or equal to a preset difference parameter threshold is judged; and when the simulation-measurement difference parameter is less than or equal to the preset difference parameter threshold, it is determined that all the contact parameters are calibrated successfully.

In some implementations, the target difference parameter includes at least one of a difference parameter between the simulated axial pressure of the electrode material and the measured axial pressure of the electrode material, a difference parameter between the simulated axial strain of the electrode material and the measured axial strain of the electrode material, or a difference parameter between a simulated compacted density parameter of the electrode material and a measured compacted density parameter of the electrode material. For example, when it is calculated based on a simulated axial pressure-axial strain curve, a measured axial pressure-axial strain curve, the simulated compacted density parameter, and the measured compacted density parameter that an average error parameter between the simulated axial pressure and the measured axial pressure is less than a preset first error threshold, that an integral difference parameter between the simulated axial strain and the measured axial strain is less than a preset second error threshold, and that an error parameter between the simulated compacted density parameter and the measured compacted density parameter is less than a preset third error threshold, it can be determined that the simulation-measurement difference parameter is less than the preset difference parameter threshold. That is, it can be determined that all the contact parameters are calibrated successfully.

It can be seen that in the preceding implementations, the target difference parameter of the electrode material can be calculated flexibly according to the simulated axial pressure-axial strain relationship of the electrode material and the measured axial pressure-axial strain relationship of the electrode material. Then the simulation-measurement difference parameter of the electrode material is calculated according to the target difference parameter, thereby implementing the judgment of whether the contact parameters are calibrated successfully. Such an arrangement can improve the reliability and accuracy of judging whether the contact parameters are calibrated successfully, thereby guaranteeing that the establishment of the calibration model can provide a good theoretical basis for the subsequent preparation process of the electrode material, and thus providing appropriate parameters for the compaction forming process of the electrode material.

### Embodiment three

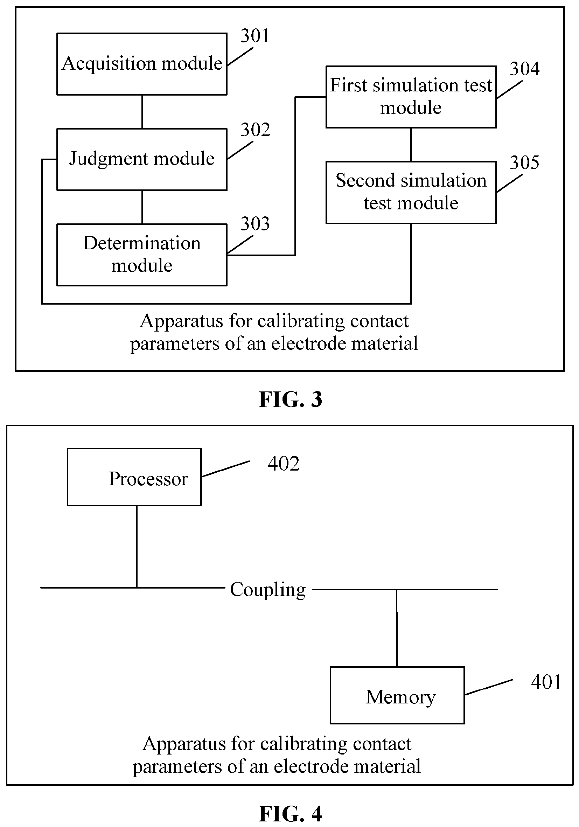

Referring to FIG. 3, FIG. 3 is a structural diagram of an apparatus for calibrating contact parameters of electrode materials according to an embodiment of the present disclosure. As shown in FIG. 3, the apparatus for calibrating contact parameters of electrode materials may include an acquisition module 301, a judgment module 302, a determination module 303, a first simulation test module 304, and a second simulation test module 305.

The acquisition module 301 is configured to acquire an axial force-displacement curve of a target pellet of an electrode material.

The judgment module 302 is configured to judge, according to the axial force-displacement curve, whether the electrode material meets an adaptation condition of a preset contact model.

The determination module 303 is configured to determine calibration ranges of a plurality of contact parameters of the electrode material when the electrode material meets the adaptation condition of the preset contact model.

The first simulation test module 304 is configured to perform a discrete element simulation test on the electrode material based on the calibration ranges of all the contact parameters and a preset uniaxial confined compression calibration model to obtain a target discrete element simulation parameter of the electrode material.

The second simulation test module 305 is configured to perform a uniaxial confined compression simulation test on the electrode material according to the target discrete element simulation parameter to obtain a simulated axial pressure-axial strain relationship of the electrode material.

The judgment module 302 is further configured to judge whether all the contact parameters are calibrated successfully according to the simulated axial pressure-axial strain relationship, a pre-obtained measured axial pressure-axial strain relationship of the electrode material, and a measured compacted density parameter.

The determination module 303 is further configured to determine calibration results of all the contact parameters according to the target discrete element simulation parameter when all the plurality of contact parameters are calibrated successfully.

In the embodiment of the present disclosure, the axial force-displacement curve is obtained by performing a uniaxial unconfined compression test on the target pellet. The uniaxial confined compression calibration model is established based on the contact model and an actual application scenario of the electrode material.

It can be seen that the apparatus for calibrating contact parameters of electrode materials described in FIG. 3 can be implemented through adopting a test-simulation joint calibration method to calibrate the contact parameters of the electrode material. Such an arrangement simplifies the testing process and improves the reliability and accuracy of calibrating the contact parameters of the electrode material compared with the direct measurement method, thereby providing a good theoretical basis for the preparation process of the electrode material.

In some implementations, the manner in which the determination module 303 determines the calibration ranges of the contact parameters of the electrode material specifically includes the steps below.

A particle density parameter of the electrode material is determined, and a particle diameter distribution parameter of the electrode material is determined.

The calibration ranges of the contact parameters of the electrode material are determined according to the particle density parameter and the particle diameter distribution parameter.

In some implementations, the particle diameter distribution parameter includes a particle diameter parameter and a particle proportion parameter corresponding to the particle diameter parameter. All the contact parameters include at least one of an inter-particle recovery coefficient, an inter-particle static friction coefficient, an inter-particle rolling friction coefficient, a shear modulus coefficient, a damping coefficient, a stiffness factor parameter, or a yield strength parameter.

It can be seen that in the implementations of the apparatus for calibrating contact parameters of electrode materials described in FIG. 3, the particle density parameter of the electrode material and the particle diameter distribution parameter of the electrode material can be measured based on the tapping method and the sieving method. Then the calibration ranges of the contact parameters of the electrode material can be determined. Such an arrangement can provide a reliable simulation parameter reference range for the subsequent discrete element simulation test, improving the reliability, accuracy and effectiveness of performing the discrete element simulation test, thereby improving the reliability and accuracy of comparing the subsequent test-simulation results, and thus improving the accuracy of judging whether the contact parameters of the electrode material are calibrated successfully.

In some implementations, the manner in which the first simulation test module 304 performs the discrete element simulation test on the electrode material based on the calibration ranges of all the contact parameters and the preset uniaxial confined compression calibration model to obtain the target discrete element simulation parameter of the electrode material specifically includes the steps below.

A compression molding simulation test is performed on the electrode material based on the calibration ranges of all the contact parameters and the preset uniaxial confined compression calibration model to determine all significant contact parameters among all the contact parameters and a regression equation corresponding to all the contact parameters.

A steepest ascent test is performed on all the significant contact parameters according to calibration ranges of all the significant contact parameters and the regression equation to obtain steepest ascent test results corresponding to all the significant contact parameters.

A response surface test is performed on all the significant contact parameters according to the steepest ascent test results to obtain response surface test results corresponding to all the significant contact parameters.

The regression equation is solved based on the response surface test results to obtain the target discrete element simulation parameter of the electrode material.

It can be seen that in the implementations of the apparatus for calibrating contact parameters of electrode materials described in FIG. 3, the compression molding simulation test can be performed on the electrode material through the uniaxial confined compression calibration model to obtain the significant contact parameters of the electrode material and the regression equation corresponding to the contact parameters. Then the steepest ascent test and the response surface test can be performed on the significant contact parameters of the electrode material so as to solve the regression equation and obtain the optimal solutions of the contact parameters of the electrode material. Such an arrangement can guarantee the reliability and accuracy of the simulation result of the model and the operation stability of the model, thereby improving the calibration efficiency of the contact parameters of the electrode material and thus applying the calibration model to the preparation process of the electrode material.

In some implementations, the manner in which the first simulation test module 304 performs the compression molding simulation test on the electrode material based on the calibration ranges of all the contact parameters and the preset uniaxial confined compression calibration model to determine all the significant contact parameters among all the contact parameters and the regression equation corresponding to all the contact parameters specifically includes the steps below.

The compression molding simulation test is performed on the electrode material based on the calibration ranges of all the contact parameters and the preset uniaxial confined compression calibration model by taking an axial strain rate of the electrode material as a response index to obtain an axial strain rate simulation parameter of the electrode material regarding all the contact parameters and take the axial strain rate simulation parameter as a compression molding simulation result of the electrode material.

Parameter screening test significance analysis is performed on all the contact parameters according to the compression molding simulation result to obtain a significance parameter of each contact parameter; and all the significant contact parameters whose significance parameters are greater than or equal to a preset significance threshold are determined from all the contact parameters according to significance parameters of all the contact parameters.

The regression equation corresponding to all the contact parameters at the axial strain rate is determined according to the compression molding simulation result.

It can be seen that in the implementations of the apparatus for calibrating contact parameters of electrode materials described in FIG. 3, uniaxial confined compression can be taken as the calibration model and applied to the simulation of the compaction forming process of the electrode material. Then the significant contact parameters of the electrode material and the regression equation corresponding to the contact parameters are determined. Such an arrangement helps improve the reliability and accuracy of determining the significant contact parameters and the regression equation, thus improving the efficiency of performing the subsequent steepest ascent test on the significant contact parameters and obtaining the optimal solutions of the contact parameters accurately and in rapid convergence.

In some implementations, the manner in which the first simulation test module 304 performs the steepest ascent test on all the significant contact parameters according to the calibration ranges of all the significant contact parameters and the regression equation to obtain the steepest ascent test results corresponding to all the significant contact parameters includes the steps below.

Analysis is performed on an influence effect of the most significant contact parameter with the largest significance parameter at the axial strain rate according to the regression equation.

A climbing unit and a climbing gradient direction that correspond to each significant contact parameter are determined according to the influence effect of the most significant contact parameter and a calibration range corresponding to the most significant contact parameter.

The steepest ascent test is performed on all the significant contact parameters according to climbing units corresponding to all the significant contact parameters, climbing gradient directions corresponding to all the significant contact parameters, and the calibration ranges corresponding to all the significant contact parameters to obtain the steepest ascent test results corresponding to all the significant contact parameters.

It can be seen that in the implementations of the apparatus for calibrating contact parameters of electrode materials described in FIG. 3, the most significant contact parameter can be taken as the climbing unit and the climbing gradient direction so as to perform the steepest ascent test on all the significant contact parameters to obtain the steepest ascent test results corresponding to all the significant contact parameters. Such an arrangement helps improve the reliability and accuracy of performing the steepest ascent test on the significant contact parameters, thereby improving the reliability and accuracy of performing the subsequent response surface test on the significant contact parameters, and thus improving the performing solving operation on efficiency of the target discrete element simulation parameter of the electrode material.

In some implementations, the manner in which the first simulation test module 304 performs the response surface test on all the significant contact parameters according to the steepest ascent test results to obtain the response surface test results corresponding to all the significant contact parameters includes the steps below.

The measured axial pressure-axial strain relationship of the electrode material is acquired.

A response surface test parameter range corresponding to each significant contact parameter is determined according to the steepest ascent test results; and the response surface test is performed on all the significant contact parameters according to response surface test parameter ranges corresponding to all the significant contact parameters and preset variable research objects corresponding to all the significant contact parameters by taking the axial strain rate and the relationship value as response indexes to obtain the response surface test results corresponding to all the significant contact parameters.

In some implementations, the measured axial pressure-axial strain relationship is obtained by performing a uniaxial confined compression test. The measured axial pressure-axial strain relationship includes a relationship value between the measured axial pressure and measured axial strain that are applied to the electrode material in the uniaxial confined compression test. The variable research objects include at least one of a single variable research object, an interactive variable research object, or a square variable research object.

It can be seen that in the implementations of the apparatus for calibrating contact parameters of electrode materials described in FIG. 3, the response surface test parameter ranges corresponding to the significant contact parameters can be determined according to the steepest ascent test results. Then the response surface test is performed on the significant contact parameters according to the response surface test parameter ranges corresponding to the significant contact parameters and the preset variable research objects corresponding to the significant contact parameters by taking the axial strain rate and the relationship value as the response indexes. Such an arrangement improves the reliability and accuracy of performing the response surface test on the significant contact parameters, thereby improving the efficiency of performing solving operation on the regression equation subsequently, and thus improving the efficiency and accuracy of calibrating the contact parameters of the electrode material.

In another optional embodiment, the manner in which the judgment module 302 determines, according to the simulated axial pressure-axial strain relationship, the pre-obtained measured axial pressure-axial strain relationship of the electrode material, and the measured compacted density parameter, whether all the contact parameters are calibrated successfully specifically includes the steps below.

A target difference parameter of the electrode material is calculated according to the simulated axial pressure-axial strain relationship, the pre-obtained measured axial pressure-axial strain relationship of the electrode material, and the measured compacted density parameter.

A simulation-measurement difference parameter of the electrode material is determined according to the target difference parameter; whether the simulation-measurement difference parameter is less than or equal to a preset difference parameter threshold is judged; and when the simulation-measurement difference parameter is less than or equal to the preset difference parameter threshold, it is determined that all the contact parameters are calibrated successfully.

In some implementations, the target difference parameter includes at least one of a difference parameter between the simulated axial pressure of the electrode material and the measured axial pressure of the electrode material, a difference parameter between the simulated axial strain of the electrode material and the measured axial strain of the electrode material, or a difference parameter between a simulated compacted density parameter of the electrode material and a measured compacted density parameter of the electrode material.

It can be seen that in the implementations of the apparatus for calibrating contact parameters of electrode materials described in FIG. 3, the target difference parameter of the electrode material can be calculated flexibly according to the simulated axial pressure-axial strain relationship of the electrode material and the measured axial pressure-axial strain relationship of the electrode material. Then the simulation-measurement difference parameter of the electrode material is calculated according to the target difference parameter, thereby implementing the judgment of whether the contact parameters are calibrated successfully. Such an arrangement can improve the reliability and accuracy of judging whether the contact parameters are calibrated successfully, thereby guaranteeing that the establishment of the calibration model can provide a good theoretical basis for the subsequent preparation process of the electrode material, and thus providing appropriate parameters for the compaction forming process of the electrode material.

### Embodiment four

Referring to FIG. 4, FIG. 4 is a structural diagram of an apparatus for calibrating contact parameters of electrode materials according to an embodiment of the present disclosure. As shown in FIG. 4, the apparatus for calibrating contact parameters of electrode materials may include a memory 401 storing executable program codes and a processor 402 coupled with the memory 401.

The processor 402 calls the executable program codes stored in the memory 401 to perform steps in the method for calibrating contact parameters of an electrode material described in embodiment one of the present disclosure or embodiment two of the present disclosure.

### Embodiment five

An embodiment of the present disclosure discloses a computer storage medium for storing computer instructions. When the computer instructions are called, steps in the method for calibrating contact parameters of an electrode material described in embodiment one of the present disclosure or embodiment two of the present disclosure are performed.

### Embodiment six

An embodiment of the present disclosure discloses a computer program product. The computer program product includes a non-transitory computer-readable storage medium storing a computer program. The computer program is operable to cause a computer to perform steps in the method of calibrating contact parameters of an electrode material described in embodiment one or embodiment two.

The apparatus embodiment is described illustratively. Modules described as separate components in the apparatus embodiment may or may not be physically separated. Components presented as modules in the apparatus embodiment may or may not be physical modules, that is, may be located in one place or may be distributed over multiple network modules. Part or all of these modules may be selected according to practical requirements to achieve the object of the solution of the embodiment. Those of ordinary skills in the art can achieve understanding and implementation without creative work.

From the detailed description of the embodiments described above, it will be apparent to those skilled in the art that the method of any embodiment described above may be implemented by means of software plus a necessary general-purpose hardware platform, or may of course be implemented by hardware. Based on this understanding, the preceding technical solutions substantially, or the part contributing to the existing art, may be embodied in the form of a software product. The computer software product may be stored in a computer-readable storage medium. The storage medium includes a read-only memory (ROM), a random access memory (RAM), a programmable read-only memory (PROM), an erasable programmable read-only memory (EPROM), a one-time programmable read-only memory (OTPROM), an electrically-erasable programmable read-only memory (EEPROM), a compact disc read-only memory (CD-ROM), or other optical disc memories, magnetic disc memories, magnetic tape memories, or any other computer-readable medium capable of carrying or storing data.

## Claims

1. A method for calibrating contact parameters of electrode materials comprising:
acquiring (101) an axial force-displacement curve of a target pellet of an electrode material and judging, according to the axial force-displacement curve, whether the electrode material meets an adaptation condition of a preset contact model, wherein the axial force-displacement curve is obtained by performing a uniaxial unconfined compression test on the target pellet;
when the electrode material meets the adaptation condition of the preset contact model, determining (102) calibration ranges of a plurality of contact parameters of the electrode material and performing a discrete element simulation test on the electrode material based on the calibration ranges of all the plurality of contact parameters and a preset uniaxial confined compression calibration model to obtain a target discrete element simulation parameter of the electrode material, wherein the uniaxial confined compression calibration model is established based on the contact model and an actual application scenario of the electrode material; and
performing (103) a uniaxial confined compression simulation test on the electrode material according to the target discrete element simulation parameter to obtain a simulated axial pressure-axial strain relationship of the electrode material; judging, according to the simulated axial pressure-axial strain relationship, a pre-obtained measured axial pressure-axial strain relationship of the electrode material, and a measured compacted density parameter, whether all the plurality of contact parameters are calibrated successfully; and when all the plurality of contact parameters are calibrated successfully, determining calibration results of all the plurality of contact parameters according to the target discrete element simulation parameter.

2. The method for calibrating contact parameters of electrode materials according to claim 1, wherein determining the calibration ranges of the plurality of contact parameters of the electrode material comprises:
determining a particle density parameter of the electrode material and determining a particle diameter distribution parameter of the electrode material, wherein the particle diameter distribution parameter comprises a particle diameter parameter and a particle proportion parameter corresponding to the particle diameter parameter; and
determining the calibration ranges of the plurality of contact parameters of the electrode material according to the particle density parameter and the particle diameter distribution parameter, wherein all the plurality of contact parameters comprise at least one of: an inter-particle recovery coefficient, an inter-particle static friction coefficient, an inter-particle rolling friction coefficient, a shear modulus coefficient, a damping coefficient, a stiffness factor parameter, or a yield strength parameter.

3. The method for calibrating contact parameters of electrode materials according to claim 1, wherein performing the discrete element simulation test on the electrode material based on the calibration ranges of all the plurality of contact parameters and the preset uniaxial confined compression calibration model to obtain the target discrete element simulation parameter of the electrode material comprises:
performing a compression molding simulation test on the electrode material based on the calibration ranges of all the plurality of contact parameters and the preset uniaxial confined compression calibration model to determine all significant contact parameters among all the plurality of contact parameters and a regression equation corresponding to all the plurality of contact parameters;
performing a steepest ascent test on all the significant contact parameters according to calibration ranges of all the significant contact parameters and the regression equation to obtain steepest ascent test results corresponding to all the significant contact parameters;
performing a response surface test on all the significant contact parameters according to the steepest ascent test results to obtain response surface test results corresponding to all the significant contact parameters; and
performing solving operation on the regression equation based on the response surface test results to obtain the target discrete element simulation parameter of the electrode material.

4. The method for calibrating contact parameters of electrode materials according to claim 3, wherein performing the compression molding simulation test on the electrode material based on the calibration ranges of all the plurality of contact parameters and the preset uniaxial confined compression calibration model to determine all the significant contact parameters among all the plurality of contact parameters and the regression equation corresponding to all the plurality of contact parameters comprises:
performing, based on the calibration ranges of all the plurality of contact parameters and the preset uniaxial confined compression calibration model, the compression molding simulation test on the electrode material by taking an axial strain rate of the electrode material as a response index to obtain an axial strain rate simulation parameter of the electrode material under all the plurality of contact parameters, and the axial strain rate simulation parameter serving as a compression molding simulation result of the electrode material;
performing parameter screening test significance analysis on all the plurality of contact parameters according to the compression molding simulation result to obtain a significance parameter of each of the plurality of contact parameters and determining, according to significance parameters of all the plurality of contact parameters, from all the plurality of contact parameters, all the significant contact parameters whose significance parameters are greater than or equal to a preset significance threshold; and
determining, according to the compression molding simulation result, the regression equation corresponding to all the plurality of contact parameters at the axial strain rate.

5. The method for calibrating contact parameters of electrode materials according to claim 4, wherein performing the steepest ascent test on all the significant contact parameters according to the calibration ranges of all the significant contact parameters and the regression equation to obtain the steepest ascent test results corresponding to all the significant contact parameters comprises:
performing analysis on an influence effect of a most significant contact parameter having a largest significance parameter at the axial strain rate according to the regression equation;
determining, according to the influence effect of the most significant contact parameter and a calibration range corresponding to the most significant contact parameter, a climbing unit and a climbing gradient direction that correspond to each of the significant contact parameters; and
performing the steepest ascent test on all the significant contact parameters according to climbing units corresponding to all the significant contact parameters, climbing gradient directions corresponding to all the significant contact parameters, and the calibration ranges corresponding to all the significant contact parameters to obtain the steepest ascent test results corresponding to all the significant contact parameters.

6. The method for calibrating contact parameters of electrode materials according to claim 4, wherein performing the response surface test on all the significant contact parameters according to the steepest ascent test results to obtain the response surface test results corresponding to all the significant contact parameters comprises:
acquiring the measured axial pressure-axial strain relationship of the electrode material, wherein the measured axial pressure-axial strain relationship is obtained by performing a uniaxial confined compression test on the electrode material, and the measured axial pressure-axial strain relationship comprises a relationship value between measured axial pressure and measured axial strain that are applied to the electrode material in the uniaxial confined compression test; and
determining, according to the steepest ascent test results, a response surface test parameter range corresponding to each of the significant contact parameters and performing, according to response surface test parameter ranges corresponding to all the significant contact parameters and preset variable research objects corresponding to all the significant contact parameters, the response surface test on all the significant contact parameters by taking the axial strain rate and the relationship value as response indexes to obtain the response surface test results corresponding to all the significant contact parameters, wherein the variable research objects comprise at least one of: a single variable research object, an interactive variable research object, or a square variable research object.

7. The method for calibrating contact parameters of electrode materials according to any one of claims 1 to 6, wherein judging, according to the simulated axial pressure-axial strain relationship, the pre-obtained measured axial pressure-axial strain relationship of the electrode material, and the measured compacted density parameter, whether all the plurality of contact parameters are calibrated successfully comprises:
calculating a target difference parameter of the electrode material according to the simulated axial pressure-axial strain relationship, the pre-obtained measured axial pressure-axial strain relationship of the electrode material, and the measured compacted density parameter; and
determining a simulation-measurement difference parameter of the electrode material according to the target difference parameter; judging whether the simulation-measurement difference parameter is less than or equal to a preset difference parameter threshold; and when the simulation-measurement difference parameter is less than or equal to the preset difference parameter threshold, determining that all the plurality of contact parameters are calibrated successfully.

8. The method for calibrating contact parameters of electrode materials according to claim 7, wherein the target difference parameter comprises at least one of: a difference parameter between simulated axial pressure of the electrode material and measured axial pressure of the electrode material, a difference parameter between simulated axial strain of the electrode material and measured axial strain of the electrode material, or a difference parameter between a simulated compacted density parameter of the electrode material and a measured compacted density parameter of the electrode material.

9. An apparatus for calibrating contact parameters of electrode materials comprising:
a memory (401) storing executable program codes; and
a processor (402) coupled with the memory (401);
wherein the processor (402) calls the executable program codes stored in the memory (401) to perform the method for calibrating contact parameters of electrode materials according to any one of claims 1 to 8.

10. A computer storage medium for storing computer instructions, wherein when the computer instructions are called, the method for calibrating contact parameters of electrode materials according to any one of claims 1 to 8 is performed.

## Patentansprüche

1. Verfahren zur Kalibrierung von Kontaktparametern von Elektrodenmaterialien, umfassend:
Erfassen (101) einer Axialkraft-Verschiebungskurve eines Zielpresslings eines Elektrodenmaterials und Beurteilen gemäß der Axialkraft-Verschiebungskurve, ob das Elektrodenmaterial eine Anpassungsbedingung eines vorgegebenen Kontaktmodells erfüllt, wobei die Axialkraft-Verschiebungskurve durch Durchführen eines einaxialen unbegrenzten Druckversuchs an dem Zielpressling erhalten wird;
wenn das Elektrodenmaterial die Anpassungsbedingung des vorgegebenen Kontaktmodells erfüllt, Bestimmen (102) von Kalibrierbereichen einer Vielzahl von Kontaktparametern des Elektrodenmaterials und Durchführen eines Diskrete-Elemente-Simulationsversuchs an dem Elektrodenmaterial basierend auf den Kalibrierbereichen der gesamten Vielzahl von Kontaktparametern und einem vorgegebenen Kalibriermodell für einaxialen begrenzten Druck, um einen Diskrete-Elemente-Simulationszielparameter des Elektrodenmaterials zu erhalten, wobei das Kalibriermodell für einaxialen begrenzten Druck basierend auf dem Kontaktmodell und einem tatsächlichen Anwendungsszenario des Elektrodenmaterials festgelegt wird, und
Durchführen (103) eines einaxialen begrenzten Drucksimulationsversuchs an dem Elektrodenmaterial gemäß dem Diskrete-Elemente-Simulationszielparameter, um eine simulierte Beziehung zwischen axialem Druck und axialer Dehnung des Elektrodenmaterials zu erhalten; Beurteilen gemäß der simulierten Beziehung zwischen axialem Druck und axialer Dehnung einer zuvor erhaltenen gemessenen Beziehung zwischen axialem Druck und axialer Dehnung des Elektrodenmaterials und einem gemessenen Verdichtungsdichteparameter, ob die gesamte Vielzahl von Kontaktparametern erfolgreich kalibriert ist, und Bestimmen von Kalibrierergebnissen der gesamten Vielzahl von Kontaktparametern gemäß dem Diskrete-Elemente-Simulationszielparameter, wenn die gesamte Vielzahl von Kontaktparametern erfolgreich kalibriert ist.

2. Verfahren zur Kalibrierung von Kontaktparametern von Elektrodenmaterialien nach Anspruch 1, wobei das Bestimmen der Kalibrierbereiche der Vielzahl von Kontaktparametern des Elektrodenmaterials Folgendes umfasst:
Bestimmen eines Partikeldichteparameters des Elektrodenmaterials und Bestimmen eines Partikeldurchmesserverteilungsparameters des Elektrodenmaterials, wobei der Partikeldurchmesserverteilungsparameter einen Partikeldurchmesserparameter und einen dem Partikeldurchmesserparameter entsprechenden Partikelanteilsparameter umfasst, und
Bestimmen der Kalibrierbereiche der Vielzahl von Kontaktparametern des Elektrodenmaterials gemäß dem Partikeldichteparameter und dem Partikeldurchmesserverteilungsparameter, wobei die gesamte Vielzahl von Kontaktparametern mindestens einen der folgenden Werte umfasst: einen Partikel-Partikel-Rückprallkoeffizienten, einen Partikel-Partikel-Haftreibungskoeffizienten, einen Partikel-Partikel-Rollreibungskoeffizienten, einen Schermodulkoeffizienten, einen Dämpfungskoeffizienten, einen Steifigkeitsfaktorparameter oder einen Fließgrenzenparameter.

3. Verfahren zur Kalibrierung von Kontaktparametern von Elektrodenmaterialien nach Anspruch 1, wobei das Durchführen des Diskrete-Elemente-Simulationsversuchs an dem Elektrodenmaterial basierend auf den Kalibrierbereichen der gesamten Vielzahl von Kontaktparametern und dem vorgegebenen Kalibriermodell für einaxialen begrenzten Druck, um den Diskrete-Elemente-Simulationszielparameter des Elektrodenmaterials zu erhalten, Folgendes umfasst:
Durchführen eines Druckformungssimulationsversuchs an dem Elektrodenmaterial basierend auf den Kalibrierbereichen der gesamten Vielzahl von Kontaktparametern und dem vorgegebenen Kalibriermodell für einaxialen begrenzten Druck, um alle signifikanten Kontaktparameter aus der gesamten Vielzahl von Kontaktparametern sowie eine der gesamten Vielzahl von Kontaktparametern entsprechende Regressionsgleichung zu bestimmen;
Durchführen eines Versuchs des steilsten Anstiegs für alle signifikanten Kontaktparameter gemäß den Kalibrierbereichen aller signifikanten Kontaktparameter und der Regressionsgleichung, um allen signifikanten Kontaktparametern entsprechende Ergebnisse des Versuchs des steilsten Anstiegs zu erhalten;
Durchführen eines Antwortflächenversuchs für alle signifikanten Kontaktparameter gemäß den Ergebnissen des Versuchs des steilsten Anstiegs, um allen signifikanten Kontaktparametern entsprechende Ergebnisse des Antwortflächenversuchs zu erhalten, und
Durchführen eines Lösungsvorgangs für die Regressionsgleichung basierend auf den Ergebnissen des Antwortflächenversuchs, um den Diskrete-Elemente-Simulationszielparameter des Elektrodenmaterials zu erhalten.

4. Verfahren zur Kalibrierung von Kontaktparametern von Elektrodenmaterialien nach Anspruch 3, wobei das Durchführen des Druckformungssimulationsversuchs an dem Elektrodenmaterial basierend auf den Kalibrierbereichen der gesamten Vielzahl von Kontaktparametern und dem vorgegebenen Kalibriermodell für einaxialen begrenzten Druck, um alle signifikanten Kontaktparameter aus der gesamten Vielzahl von Kontaktparametern sowie die der gesamten Vielzahl von Kontaktparametern entsprechende Regressionsgleichung zu bestimmen, Folgendes umfasst:
basierend auf den Kalibrierbereichen der gesamten Vielzahl von Kontaktparametern und dem vorgegebenen Kalibriermodell für einaxialen begrenzten Druck Durchführen des Druckformungssimulationsversuchs an dem Elektrodenmaterial, indem eine axiale Dehnungsrate des Elektrodenmaterials als Antwortindex herangezogen wird, um einen Axialdehnungsraten-Simulationsparameter des Elektrodenmaterials unter der gesamten Vielzahl von Kontaktparametern zu erhalten, wobei der Axialdehnungsraten-Simulationsparameter als Druckformungssimulationsergebnis des Elektrodenmaterials dient;
Durchführen einer Signifikanzanalyse eines Parameterscreening-Versuchs für die gesamte Vielzahl von Kontaktparametern gemäß dem Druckformungssimulationsergebnis, um einen Signifikanzparameter eines jeden der Vielzahl von Kontaktparametern zu erhalten, und Bestimmen gemäß den Signifikanzparametern der gesamten Vielzahl von Kontaktparametern aus der gesamten Vielzahl von Kontaktparametern aller signifikanten Kontaktparameter, deren Signifikanzparameter größer oder gleich einem vorgegebenen Signifikanzschwellenwert sind, und
Bestimmen der der gesamten Vielzahl von Kontaktparametern entsprechenden Regressionsgleichung für die Axialdehnungsrate gemäß dem Druckformungssimulationsergebnis.

5. Verfahren zur Kalibrierung von Kontaktparametern von Elektrodenmaterialien nach Anspruch 4, wobei das Durchführen des Versuchs des steilsten Anstiegs für alle signifikanten Kontaktparameter gemäß den Kalibrierbereichen aller signifikanten Kontaktparameter und der Regressionsgleichung, um allen signifikanten Kontaktparametern entsprechende Ergebnisse des Versuchs des steilsten Anstiegs zu erhalten, Folgendes umfasst:
Durchführen einer Analyse der Einflusswirkung eines signifikantesten Kontaktparameters, aufweisend den größten Signifikanzparameter, auf die axiale Dehnungsrate gemäß der Regressionsgleichung;
Bestimmen einer Steigeinheit und einer Steiggradientenrichtung, die jeweils den signifikanten Kontaktparametern entsprechen, gemäß der Einflusswirkung des signifikantesten Kontaktparameters und einem dem signifikantesten Kontaktparameter entsprechenden Kalibrierbereich, und
Durchführen des Versuchs des steilsten Anstiegs für alle signifikanten Kontaktparameter gemäß allen signifikanten Kontaktparametern entsprechenden Steigeinheiten, allen signifikanten Kontaktparametern entsprechenden Steiggradientenrichtungen und den allen signifikanten Kontaktparametern entsprechenden Kalibrierbereichen, um allen signifikanten Kontaktparametern entsprechende Ergebnisse des Versuchs des steilsten Anstiegs zu erhalten.

6. Verfahren zur Kalibrierung von Kontaktparametern von Elektrodenmaterialien nach Anspruch 4, wobei das Durchführen des Antwortflächenversuchs für alle signifikanten Kontaktparameter gemäß den Ergebnissen des Versuchs des steilsten Anstiegs, um allen signifikanten Kontaktparametern entsprechende Ergebnisse des Antwortflächenversuchs zu erhalten, Folgendes umfasst:
Erfassen der gemessenen Beziehung zwischen axialem Druck und axialer Dehnung des Elektrodenmaterials, wobei die gemessene Beziehung zwischen axialem Druck und axialer Dehnung durch Durchführen eines einaxialen begrenzten Druckversuchs an dem Elektrodenmaterial erhalten wird und die gemessene Beziehung zwischen axialem Druck und axialer Dehnung einen Beziehungswert zwischen gemessenem axialem Druck und gemessener axialer Dehnung umfasst, die bei dem einaxialen begrenzten Druckversuch auf das Elektrodenmaterial angewendet werden, und
Bestimmen eines jedem der signifikanten Kontaktparameter entsprechenden Antwortflächenversuchsparameterbereichs gemäß den Ergebnissen des Versuchs des steilsten Anstiegs und Durchführen des Antwortflächenversuchs für alle signifikanten Kontaktparameter gemäß den allen signifikanten Kontaktparametern entsprechenden Antwortflächenversuchsparameterbereichen und allen signifikanten Kontaktparametern entsprechenden vorgegebenen Variablen-Untersuchungsobjekten, indem die axiale Dehnungsrate und der Beziehungswert als Antwortindizes herangezogen werden, um die allen signifikanten Kontaktparametern entsprechenden Antwortflächenversuchsergebnisse zu erhalten, wobei die Variablen-Untersuchungsobjekte mindestens eines der folgenden Untersuchungsobjekte umfassen: ein Einzelvariablen-Untersuchungsobjekt, ein Interaktionsvariablen-Untersuchungsobjekt oder ein quadratisches Variablen-Untersuchungsobjekt.

7. Verfahren zur Kalibrierung von Kontaktparametern von Elektrodenmaterialien nach einem der Ansprüche 1 bis 6, wobei das Beurteilen gemäß der simulierten Beziehung zwischen axialem Druck und axialer Dehnung, der zuvor erhaltenen gemessenen Beziehung zwischen axialem Druck und axialer Dehnung des Elektrodenmaterials und dem gemessenen Verdichtungsdichteparameter, ob die gesamte Vielzahl von Kontaktparametern erfolgreich kalibriert ist, Folgendes umfasst:
Berechnen eines Zieldifferenzparameters des Elektrodenmaterials gemäß der simulierten Beziehung zwischen axialem Druck und axialer Dehnung, der zuvor erhaltenen gemessenen Beziehung zwischen axialem Druck und axialer Dehnung des Elektrodenmaterials und dem gemessenen Verdichtungsdichteparameter und
Bestimmen eines Simulationsmessungsdifferenzparameters des Elektrodenmaterials gemäß dem Zieldifferenzparameter; Beurteilen, ob der Simulationsmessungsdifferenzparameter kleiner oder gleich einem vorgegebenen Differenzparameterschwellenwert ist und Bestimmen, dass die gesamte Vielzahl von Kontaktparametern erfolgreich kalibriert ist, wenn der Simulationsmessungsdifferenzparameter kleiner oder gleich dem vorgegebenen Differenzparameterschwellenwert ist.

8. Verfahren zur Kalibrierung von Kontaktparametern von Elektrodenmaterialien nach Anspruch 7, wobei der Zieldifferenzparameter mindestens einen der folgenden Parameter umfasst: einen Differenzparameter zwischen simuliertem axialem Druck des Elektrodenmaterials und gemessenem axialem Druck des Elektrodenmaterials, einen Differenzparameter zwischen simulierter axialer Dehnung des Elektrodenmaterials und gemessener axialer Dehnung des Elektrodenmaterials oder einen Differenzparameter zwischen einem simulierten Verdichtungsdichteparameter des Elektrodenmaterials und einem gemessenen Verdichtungsdichteparameter des Elektrodenmaterials.

9. Vorrichtung zur Kalibrierung von Kontaktparametern von Elektrodenmaterialien, umfassend:
einen Speicher (401), in dem ausführbare Programmcodes gespeichert sind, und
einen Prozessor (402), der mit dem Speicher (401) gekoppelt ist,
wobei der Prozessor (402) die in dem Speicher (401) gespeicherten ausführbaren Programmcodes abruft, um das Verfahren zur Kalibrierung von Kontaktparametern von Elektrodenmaterialien nach einem der Ansprüche 1 bis 8 durchzuführen.

10. Computerspeichermedium zur Speicherung von Computeranweisungen, wobei das Verfahren zur Kalibrierung von Kontaktparametern von Elektrodenmaterialien nach einem der Ansprüche 1 bis 8 durchgeführt wird, wenn die Computeranweisungen abgerufen werden.

## Revendications

1. Procédé d'étalonnage de paramètres de contact de matériaux d'électrode, comprenant :
l'acquisition (101) d'une courbe force axiale-déplacement d'une pastille cible d'un matériau d'électrode et la détermination, en fonction de la courbe force axiale-déplacement, si le matériau d'électrode satisfait une condition d'adaptation d'un modèle de contact prédéfini, dans lequel la courbe force axiale-déplacement est obtenue en réalisant un test de compression non-confinée uniaxiale sur la pastille cible ;
lorsque le matériau d'électrode satisfait la condition d'adaptation du modèle de contact prédéfini, la détermination (102) de plages d'étalonnage d'une pluralité de paramètres de contact du matériau d'électrode et la réalisation d'un test de simulation d'élément discret sur le matériau d'électrode sur la base des plages d'étalonnage de l'ensemble des paramètres de la pluralité de paramètres de contact et d'un modèle d'étalonnage de compression confinée uniaxiale prédéfini pour obtenir un paramètre de simulation d'élément discret cible du matériau d'électrode, dans lequel le modèle d'étalonnage de compression confinée uniaxiale est établi sur la base du modèle de contact et d'un scénario d'application réel du matériau d'électrode ; et
la réalisation (103) d'un test de simulation de compression confinée uniaxiale sur le matériau d'électrode en fonction du paramètre de simulation d'élément discret cible pour obtenir une relation pression axiale-contrainte axiale simulée du matériau d'électrode ; la détermination, en fonction de la relation pression axiale-contrainte axiale simulée, d'une relation pression axiale-contrainte axiale mesurée pré-obtenue du matériau d'électrode, et d'un paramètre de densité compactée mesuré, si l'ensemble des paramètres de la pluralité de paramètres de contact sont étalonnés avec succès ; et lorsque l'ensemble des paramètres de la pluralité de paramètres de contact sont étalonnés avec succès, la détermination de résultats d'étalonnage de l'ensemble des paramètres de la pluralité de paramètres de contact en fonction du paramètre de simulation d'élément discret cible.

2. Procédé d'étalonnage de paramètres de contact de matériaux d'électrode selon la revendication 1, dans lequel la détermination des plages d'étalonnage de la pluralité de paramètres de contact du matériau d'électrode comprend :
la détermination d'un paramètre de densité de particules du matériau d'électrode et la détermination d'un paramètre de distribution de diamètre de particule du matériau d'électrode, dans lequel le paramètre de distribution de diamètre de particule comprend un paramètre de diamètre de particule et un paramètre de proportion de particule correspondant au paramètre de diamètre de particule ; et
la détermination des plages d'étalonnage de la pluralité de paramètres de contact du matériau d'électrode en fonction du paramètre de densité de particules et du paramètre de distribution de diamètre de particule, dans lequel l'ensemble des paramètres de la pluralité de paramètres de contact comprennent au moins l'un parmi : un coefficient de récupération inter-particules, un coefficient de frottement statique inter-particules, un coefficient de frottement roulant inter-particules, un coefficient de module de cisaillement, un coefficient d'amortissement, un paramètre de facteur de rigidité, ou un paramètre de limite d'élasticité.

3. Procédé d'étalonnage de paramètres de contact de matériaux d'électrode selon la revendication 1, dans lequel la réalisation du test de simulation d'élément discret sur le matériau d'électrode sur la base des plages d'étalonnage de l'ensemble des paramètres de la pluralité de paramètres de contact et du modèle d'étalonnage de compression confinée uniaxiale prédéfini pour obtenir le paramètre de simulation d'élément discret cible du matériau d'électrode comprend :
la réalisation d'un test de simulation de moulage par compression sur le matériau d'électrode sur la base des plages d'étalonnage de l'ensemble des paramètres de la pluralité de paramètres de contact et du modèle d'étalonnage de compression confinée uniaxiale prédéfini pour déterminer tous les paramètres de contact significatifs parmi l'ensemble des paramètres de la pluralité de paramètres de contact et une équation de régression correspondant à l'ensemble des paramètres de la pluralité de paramètres de contact ;
la réalisation d'un test de montée la plus forte sur tous les paramètres de contact significatifs en fonction de plages d'étalonnage de tous les paramètres de contact significatifs et de l'équation de régression pour obtenir des résultats de test de montée la plus forte correspondant à tous les paramètres de contact significatifs ;
la réalisation d'un test de surface de réponse sur tous les paramètres de contact significatifs en fonction des résultats de test de montée la plus forte pour obtenir des résultats de test de surface de réponse correspondant à tous les paramètres de contact significatifs ; et
la réalisation d'une opération de résolution sur l'équation de régression sur la base des résultats de test de surface de réponse pour obtenir le paramètre de simulation d'élément discret cible du matériau d'électrode.

4. Procédé d'étalonnage de paramètres de contact de matériaux d'électrode selon la revendication 3, dans lequel la réalisation du test de simulation de moulage par compression sur le matériau d'électrode sur la base des plages d'étalonnage de l'ensemble des paramètres de la pluralité de paramètres de contact et du modèle d'étalonnage de compression confinée uniaxiale prédéfini pour déterminer tous les paramètres de contact significatifs parmi l'ensemble des paramètres de la pluralité de paramètres de contact et l'équation de régression correspondant à l'ensemble des paramètres de la pluralité de paramètres de contact comprend :
la réalisation, sur la base des plages d'étalonnage de l'ensemble des paramètres de la pluralité de paramètres de contact et du modèle d'étalonnage de compression confinée uniaxiale prédéfini, du test de simulation de moulage par compression sur le matériau d'électrode en considérant une vitesse de déformation axiale du matériau d'électrode comme indice de réponse pour obtenir un paramètre de simulation de vitesse de déformation axiale du matériau d'électrode sous l'ensemble des paramètres de la pluralité de paramètres de contact, et le paramètre de simulation de vitesse de déformation axiale servant de résultat de simulation de moulage par compression du matériau d'électrode ;
la réalisation d'une analyse de signifiance de test de criblage de paramètres sur l'ensemble des paramètres de la pluralité de paramètres de contact en fonction du résultat de simulation de moulage par compression pour obtenir un paramètre de signifiance de chacun de la pluralité de paramètres de contact et la détermination, en fonction de paramètres de signifiance de l'ensemble des paramètres de la pluralité de paramètres de contact, à partir de l'ensemble des paramètres de la pluralité de paramètres de contact, de tous les paramètres de contact significatifs dont les paramètres de signifiance sont supérieurs ou égaux à un seuil de signifiance prédéfini ; et
la détermination, en fonction du résultat de simulation de moulage par compression, de l'équation de régression correspondant à l'ensemble des paramètres de la pluralité de paramètres de contact à la vitesse de déformation axiale.

5. Procédé d'étalonnage de paramètres de contact de matériaux d'électrode selon la revendication 4, dans lequel la réalisation du test de montée la plus forte sur tous les paramètres de contact significatifs en fonction des plages d'étalonnage de tous les paramètres de contact significatifs et de l'équation de régression pour obtenir les résultats de test de montée la plus forte correspondant à tous les paramètres de contact significatifs comprend :
la réalisation d'une analyse sur un effet d'influence d'un paramètre de contact le plus significatif présentant un paramètre de signifiance le plus grand à la vitesse de déformation axiale en fonction de l'équation de régression ;
la détermination, en fonction de l'effet d'influence du paramètre de contact le plus significatif et d'une plage d'étalonnage correspondant au paramètre de contact le plus significatif, d'une unité de montée et d'une direction de gradient de montée qui correspondent à chacun des paramètres de contact significatifs ; et
la réalisation du test de montée la plus forte sur tous les paramètres de contact significatifs en fonction d'unités de montée correspondant à tous les paramètres de contact significatifs, de directions de gradient de montée correspondant à tous les paramètres de contact significatifs, et des plages d'étalonnage correspondant à tous les paramètres de contact significatifs pour obtenir les résultats de test de montée la plus forte correspondant à tous les paramètres de contact significatifs.

6. Procédé d'étalonnage de paramètres de contact de matériaux d'électrode selon la revendication 4, dans lequel la réalisation du test de surface de réponse sur tous les paramètres de contact significatifs en fonction des résultats de test de montée la plus forte pour obtenir les résultats de test de surface de réponse correspondant à tous les paramètres de contact significatifs comprend :
l'acquisition de la relation pression axiale-contrainte axiale mesurée du matériau d'électrode, dans lequel la relation pression axiale-contrainte axiale mesurée est obtenue en réalisant un test de compression confinée uniaxiale sur le matériau d'électrode, et la relation pression axiale-contrainte axiale mesurée comprend une valeur de relation entre la pression axiale mesurée et la contrainte axiale mesurée qui sont appliquées au matériau d'électrode dans le test de compression confinée uniaxiale ; et
la détermination, en fonction des résultats de test de montée la plus forte, d'une plage de paramètres de test de surface de réponse correspondant à chacun des paramètres de contact significatifs et la réalisation, en fonction de plages de paramètres de test de surface de réponse correspondant à tous les paramètres de contact significatifs et d'objets de recherche variables prédéfinis correspondant à tous les paramètres de contact significatifs, du test de surface de réponse sur tous les paramètres de contact significatifs en considérant la vitesse de déformation axiale et la valeur de relation comme indices de réponse pour obtenir les résultats de test de surface de réponse correspondant à tous les paramètres de contact significatifs, dans lequel les objets de recherche variables comprennent au moins l'un parmi : un objet de recherche variable unique, un objet de recherche variable interactif, ou un objet de recherche variable carré.

7. Procédé d'étalonnage de paramètres de contact de matériaux d'électrode selon l'une quelconque des revendications 1 à 6, dans lequel la détermination si l'ensemble des paramètres de la pluralité de paramètres de contact sont étalonnés avec succès, en fonction de la relation pression axiale-contrainte axiale simulée, de la relation pression axiale-contrainte axiale mesurée pré-obtenue du matériau d'électrode, et du paramètre de densité compactée mesuré, comprend :
le calcul d'un paramètre de différence cible du matériau d'électrode en fonction de la relation pression axiale-contrainte axiale simulée, de la relation pression axiale-contrainte axiale mesurée pré-obtenue du matériau d'électrode, et du paramètre de densité compactée mesuré ; et
la détermination d'un paramètre de différence simulation-mesure du matériau d'électrode en fonction du paramètre de différence cible ; la détermination si le paramètre de différence simulation-mesure est inférieur ou égal à un seuil de paramètre de différence prédéfini ; et lorsque le paramètre de différence simulation-mesure est inférieur ou égal au seuil de paramètre de différence prédéfini, la détermination que l'ensemble des paramètres de la pluralité de paramètres de contact sont étalonnés avec succès.

8. Procédé d'étalonnage de paramètres de contact de matériaux d'électrode selon la revendication 7, dans lequel le paramètre de différence cible comprend au moins l'un parmi : un paramètre de différence entre une pression axiale simulée du matériau d'électrode et une pression axiale mesurée du matériau d'électrode, un paramètre de différence entre une contrainte axiale simulée du matériau d'électrode et une contrainte axiale mesurée du matériau d'électrode, ou un paramètre de différence entre un paramètre de densité compactée simulé du matériau d'électrode et un paramètre de densité compactée mesuré du matériau d'électrode.

9. Appareil d'étalonnage de paramètres de contact de matériaux d'électrode, comprenant :
une mémoire (401) stockant des codes de programme exécutables ; et
un processeur (402) couplé à la mémoire (401) ;
dans lequel le processeur (402) appelle les codes de programme exécutables stockés dans la mémoire (401) pour réaliser le procédé d'étalonnage de paramètres de contact de matériaux d'électrode selon l'une quelconque des revendications 1 à 8.

10. Support de stockage informatique pour le stockage d'instructions informatiques, dans lequel, lorsque les instructions informatiques sont appelées, le procédé d'étalonnage de paramètres de contact de matériaux d'électrode selon l'une quelconque des revendications 1 à 8 est réalisé.
